# EUROPEAN PATENT SPECIFICATION

(11) **EP 2 849 767 B1**
(45) Date of publication and mention of the grant of the patent: **25.10.2017**
(21) Application number: 13790829.9
(22) Date of filing: 14.05.2013
(51) Int. Cl.: A61K 36/13, A23K 20/158, A61P 1/00, A61P 31/04

(54) **SAPONIFIED TALL OIL FATTY ACID FOR USE IN TREATMENT AND ANIMAL FEED SUPPLEMENTS AND COMPOSITIONS**
VERSEIFTE TALLÖLFETTSÄURE FÜR BEHANDLUNG UND TIERFUTTERZUSATZSTOFFE SOWIE ZUSAMMENSETZUNGEN
ACIDE GRAS DE TALLÖL SAPONIFIÉ POUR SON UTILISATION DANS EN THÉRAPIE, DANS DES SUPPLÉMENTS ALIMENTAIRES ET COMPOSITIONS POUR ANIMAUX

(30) Priority: 14.05.2012 FI 20125509
(43) Date of publication of application: 25.03.2015
(73) Proprietor: HANKKIJA OY, 05800 Hyvinkää (FI)
(72) Inventor: VUORENMAA, Juhani, FI-05800 Hyvinkää (FI); KETTUNEN, Hannele., FI-12400 Tervakoski (FI)
(74) Representative: Papula Oy
(86) International application number: PCT/FI2013/050518
(87) International publication number: WO 2013/171370

(56) References cited:
- WO-A1-02/02106
- GB-A- 955 316
- US-A- 2 423 236
- US-A- 5 460 648
- US-A- 6 020 377
- US-A1- 2011 212 217
- US-A1- 2011 212 218
- EILEEN SMITH ET AL: "Isopimaric acid fromPinus nigra shows activity against multidrug-resistant and EMRSA strains ofStaphylococcus aureus", PHYTOTHERAPY RESEARCH, vol. 19, no. 6, 1 June 2005 (2005-06-01), pages 538-542, XP55213576, ISSN: 0951-418X, DOI: 10.1002/ptr.1711
- SONIA SAVLUCHINSKE-FEIO ET AL: "Antimicrobial activity of resin acid derivatives", APPLIED MICROBIOLOGY AND BIOTECHNOLOGY, SPRINGER, BERLIN, DE, vol. 72, no. 3, 5 August 2006 (2006-08-05) , pages 430-436, XP019422080, ISSN: 1432-0614, DOI: 10.1007/S00253-006-0517-0

## Description

### FIELD OF THE INVENTION

The invention relates to a modified tall oil fatty acid which is modified by saponification, use thereof, and feed supplement and feed composition comprising said modified tall oil fatty acid.

### BACKGROUND OF THE INVENTION

Imbalances in microbial populations and growth of harmful bacteria in the digestive tract of animals can cause significant losses in animal growth and production. These imbalances manifest themselves as intestinal disorders such as diarrhea. While microbial infections of animals have been prevented by the use of e.g. antibiotics and other agents that prevent the growth of microorganisms, stricter regulations on their use are expected. Generally, there is an increasing demand for ingredients for use in animal feeding that can modulate the microbial population in the animal digestive tract but which are readily available, well tolerated and environmentally friendly.

Fractional distillation of crude tall oil, obtained as a by-product of the Kraft process of wood pulp manufacture, produces distilled tall oil (DTO) which typically comprises over 10% resin acids and less than 90% fatty acids. Further refinement of distilled tall oil produces tall oil fatty acid (TOFA), which is available in a variety of compositions differing in the fatty acids and resin acids content. Because TOFA is an inexpensive source of fatty acids, it has previously been used in animal nutrition as an energy source. For instance, GB 955316 discloses the use of alkali metal salts of tall oil fatty acids to improve weight gain and nitrogen retention in ruminant animals.

WO 02/02106 A1 discloses compositions comprising a mixture of a dietary supplement such as an agent for altering the metabolism of the human or animal and a fatty acid source such as modified tall oil or conjugated linoleic acids.

The article E. SMITH ET AL: "lsopimaric acid from Pinus nigra shows activity against multidrug-resistant and EMRSA strains of Staphylococcus aureus", PHYTOTHERAPY RESEARCH, vol. 19, no. 6, 1 June 2005, pages 538-542, discloses the activity of isopimaric acid against MDR and MRSA strains of *S. aureus* which are becoming increasingly resistant to antibiotics.

The article S. SAVLUCHINSKE-FEIO ET AL: "Antimicrobial activity of resin acid derivatives", APPLIED MICROBIOLOGY AND BIOTECHNOLOGY, val. 72, no. 3, 5 August 2006, pages 430-436, discloses antimicrobial activity of resin acid derivatives against several microorganisms.

### PURPOSE OF THE INVENTION

The purpose of the invention is to provide a new type of modified tall oil fatty acid/feed supplement for use in the prevention of growth of harmful bacteria in the animal digestive tract and/or in the prevention of intestinal disorders.

The present inventors have surprisingly found that saponification of TOFA improves the solubility of its components and resin acids in the digestive tract of an animal in particular and significantly increases its effectiveness in the prevention of growth of harmful bacteria in the animal digestive tract, in the modulation of microbial population of the animal digestive tract and/or in the prevention of intestinal disorders.

### SUMMARY

The present invention is defined by the claims and concerns a tall oil fatty acid comprising 1-10% (w/w) resin acids which is modified by saponification, a feed supplement comprising it, and a food composition comprising the feed supplement, for use in the prevention of adverse effects on the digestive tract or health of an animal caused by growth of harmful bacteria in the animal digestive tract and/or in the prevention of intestinal disorders.

### DETAILED DESCRIPTION OF THE INVENTION

FIG la The turbidity change during 8 hours of Cl. perfringens growth as a response to saponified TOFA and digested saponified TOFA concentrations l.
FIG 1b Gas production during 8 hours by *Cl. perfringens* growth as a response to saponified TOFA and digested saponified TOFA concentrations.
FIG 2a The turbidity change during 8 hours of Cl. perfringens growth as a response to saponified TOFA and dried saponified TOFA concentrations.
FIG 2b Gas production during 8 hours by *Cl. perfringens* growth as a response to saponified TOFA and dried saponified TOFA concentrations.
FIG 3a The turbidity change during 8 hours of Cl. perfringens growth as a response to saponified TOFA and test products at dose 1.
FIG 3b The turbidity change during 8 hours of Cl. perfringens growth as a response to saponified TOFA and test products at dose 2.
FIG 3c The turbidity change during 8 hours of *S. aureus* as a response to saponified TOFA and test products at dose 1.
FIG 3d The turbidity change during 8 hours of *S. aureus* as a response to saponified TOFA and test products at dose 2.
FIG 3e The turbidity change during 8 hours of *S. suis* as a response to saponified TOFA and test products at dose 1.
FIG 3f The turbidity change during 8 hours of *C S. suis* as a response to saponified TOFA and test products at dose 2.

The present invention is based on the realization that tall oil fatty acid which is modified by saponification can be used in the prevention of growth of harmful bacteria in the animal digestive tract and/or in the prevention of intestinal disorders.

The term "tall oil fatty acid" or "TOFA" should be understood as referring to a composition obtained by distillation of crude tall oil and further refinement of distilled tall oil. TOFA or TOFA which is modified by saponification typically comprises 90-98% (w/w) fatty acids. Further, TOFA or TOFA which is modified by saponification may comprise 1-10% (w/w) resin acids.

Resin acids are known to have antimicrobial, including antibacterial, properties. However, the present inventors have found that resin acids of TOFA are poorly soluble in digestive juices and tend to precipitate in the digestive tract of an animal. Therefore their effectiveness in the digestive tract is less than optimal.

The modification of TOFA improves the solubility of its components and resin acids in the digestive tract of an animal.

In this context, the term "tall oil fatty acid which is modified by saponification" or "TOFA which is modified by saponification" should be understood as referring to TOFA that is chemically modified so as to improve the solubility of its components and resin acids in the digestive tract of an animal in particular. According to the present invention, the tall oil fatty acid which is modified by saponification for use according to the present invention comprises 1-10% (w/w) of resin acids.

In one embodiment of the present invention, TOFA or TOFA which is modified by saponification comprises 2-9 % (w/w) resin acids.

In one embodiment of the present invention, TOFA or TOFA which is modified by saponification comprises 5-9% (w/w) resin acids.

In this context, the term "resin acids" should be understood as referring to a complex mixture of various acidic compounds comprised by tall oil which share the same basic skeleton including a three-fused ring. The exact composition of the resin acids present in TOFA varies e.g. according to the species of the trees the TOFA is obtained from and the processing conditions under which it is manufactured. Resin acids typically include compounds such as abietic acid, dehydroabietic acid, levopimaric acid, neoabietic acid, pimaric acid and isopimaric acid, only to mention a few.

In one embodiment of the present invention, TOFA or TOFA which is modified by saponification comprises 90-98% (w/w) of fatty acids.

Various processes for the saponification of TOFA using e.g. NaOH or CaOH are known to a person skilled in the art.

In one embodiment of the present invention, the modified TOFA, the TOFA soap, for use according to the present invention is dried. The TOFA which is modified by saponification can be dried by spray drying, drum drying or by any other known suitable drying method.

The present invention also relates to a feed supplement comprising the tall oil fatty acid comprising 1-10% resin acids and which is modified by saponification for use in the prevention of adverse effects on the digestive tract or health of an animal caused by growth of harmful bacteria and/or for prevention of intestinal disorders.

In one embodiment of the present invention, the feed supplement comprises a tall oil fatty acid which is modified by saponification and which comprises 2-9 % (w/w) resin acids.

In one embodiment of the present invention, the feed supplement comprises a tall oil fatty acid which is modified by saponification and which comprises 5-9% (w/w) resin acids.

In this context, the term "feed supplement" should be understood as referring to a composition that may be added to a feed or used as such in the feeding of animals. The feed supplement may comprise different active ingredients. The feed supplement may be added in the feed in a concentration of 0.0001 - 5 kg//ton of dry weight, preferably 0.005 - 1 kg/ton, most preferably 0.01 - 0.1 kg/ton of the dry weight of the total amount of the feed. The TOFA which is modified by saponification or the feed supplement comprising the TOFA which is modified by saponification according to the invention may be added to the feed or feed supplement as such, or it may in general be further processed as desired.

Further, the TOFA which is modified by saponification or the feed supplement comprising the TOFA which is modified by saponification according to the invention may be added to the feed or feed supplement, or it may be administered to an animal separately (i.e. not as a part of any feed composition).

In this context, the term "feed composition" or "feed" should be understood as referring to the total feed composition of an animal diet or to a part thereof, including e.g. supplemental feed, premixes and other feed compositions. The feed may comprise different active ingredients.

In one embodiment of the present invention, the feed supplement comprises TOFA which is modified by saponification and which is absorbed into a carrier material suitable for the feed composition such as sugarbeet pulp.

In one embodiment of the present invention, the feed supplement comprises TOFA which is modified by saponification and which is dried.

The present invention also relates to a feed composition comprising the feed supplement according to the invention, for use in the prevention of adverse effects on the digestive tract or health of an animal caused by growth of harmful bacteria in the animal digestive tract and/or in the prevention of intestinal disorders. In one embodiment of the present invention, the feed composition comprises the feed supplement in an amount of 0.00001 - 0.5 % (w/w), preferably 0.0005 - 0.1 % (w/w), most preferably 0.001 - 0.01 % (w/w) of the dry weight of the total amount of the feed.

In one embodiment of the present invention, the feed composition comprises the feed supplement in the amount of 0.0005 - 0.1 % (w/w) of the dry weight of the total amount of the feed.

The modified tall oil fatty acid or feed supplement according to the invention is produced by saponification. The method comprises the steps of adding a base to an aqueous TOFA solution and heating the mixture. The mixture is stirred during the heating step. The mixture is heated at a temperature of 80 - 120 °C, preferably at 85 - 95 °C, for a period of 1 - 3 hours, preferably for 2 hours.

Any base suitable for saponification, such as an alkali metal hydroxide, can be used as the base.

In one embodiment of the present invention, the base that is used is a sodium or potassium hydroxide.

In one embodiment of the present invention, the method of producing a saponified tall oil fatty acid or feed supplement further comprises a step of drying. The dying can be carried out by spray drying, drum drying or by by any other known drying method.

The invention also relates to a method of preventing the growth of harmful bacteria in the animal digestive tract and/or preventing intestinal disorders, comprising the step of administering to an animal the tall oil fatty acid which is modified by saponification according to the invention.

In this context, the term "harmful bacteria" should be understood as referring to any bacteria that is capable of affecting the digestive tract or health of an animal in an adverse manner, including competition for nutrients with the host animal. (In this context, the term "microbial population" should be understood as referring to the microorganisms that inhabit the digestive tract, including the Bacteria and Archaea domains and microscopic members of the Eukaryote domain and also intestinal parasites. The microbial population will vary for different animal species depending on e.g. the health of an animal and on environmental factors.

In this context, the term "intestinal disorder" should be understood as referring to various disorders of the digestive tract in an animal, including e.g. diarrhea and other intestinal health problems.

In this context, the term "animal" should be understood as referring to all kinds of different animals, such as monogastric animals, ruminants, fur animals, pets and aquaculture. Non-limiting examples of different animals, including offspring, are cows, beef cattle, pigs, poultry, sheep, goats, horses, foxes, dogs, cats and fish.

In one embodiment of the present invention, the TOFA which is modified by saponification is administered to an animal in an effective amount. In a further embodiment, the TOFA which is modified by saponification is administered in a therapeutically effective amount.

The present invention has a number of advantages. TOFA is a readily available, natural, low-cost and environmentally friendly material. Further, it is non-toxic and well tolerated. Subsequently, other benefits of the invention are e.g. improved animal health and productivity, higher product quality, uniformity, food and product safety. The invention also allows the production of feed compositions and supplements at low cost.

### EXAMPLES

In the following, the present invention will be described in more detail.

### EXAMPLE 1.

### Pathogen inhibition test

*Clostridium perfringens* is a pathogenic bacterium that causes necrotic enteritis in broiler chicks and other species of poultry. This experiment was conducted to study the inhibition of *Cl. perfringens* by saponified TOFA with 5 % resin acids.

The saponified TOFA was manufactured by adding 140 mg of NaOH (sodium hydroxide) to 1 gram of TOFA, adding enough water to adjust the total dry matter (TOFA) percentage of the mixture to 18-20%, heating the mixture to + 90 °C, keeping the temperature at + 90 °C for 120 minutes, during which time the mixture was gently stirred at 15 min intervals.

The efficiency of untreated and digested test compounds was tested in a *Cl. perfringens* growth inhibition test that measures both the turbidity of the clostridial culture medium as a result of increased number of bacterial cells in a unit volume of medium, and the cumulative gas production during the simulation.

Preparations of TOFA with 5% resin acids were produced:
1. Saponified TOFA with 15% dry matter content
2. Saponified and digested TOFA

*Gastrointestinal digestion of the saponified TOFA:* Part of the saponified TOFA was digested by a pepsin-HCl treatment (pH 2.5) at +37°C for 3 hours, followed by neutralization of the digesta with NaOH (pH 6.5) and the treatment with bile acids and pancreatin for additional 3 hours at +37°C. This digestion mimics the gastric and small-intestinal digestion of monogastric animals. The digestion was made to evaluate whether the products would resist the conditions of the upper gastrointestinal tract before they enter the distal intestine with higher microbial activity.

The efficiency of TOFA against the growth of *Cl. perfringens* was tested at five concentrations of the dry matter of TOFA: 0%, 0.05%, 0.01%, 0.005%, 0.001% and, 0.0005%. Each treatment-concentration combination was replicated three times.

*Simulation procedure:* The simulation was conducted in 25-ml glass bottles containing 15 ml of sterile anaerobic TSGY- media (tryptic soy broth - yeast extract media with glucose) and the bottles were enclosed with air-tight stoppers to ensure anaerobic conditions throughout the experiment. At the beginning of the simulation 0.1 % inoculums of the overnight grown *Cl. perfringens* culture was injected to TSGY-bottles. Test compounds, or sterile deionized water for the control treatment, were added in a 150 µl final volume from the respective stock solution according to the treatment. The simulation bottles were randomized to avoid artificial bias between treatments. The bottles were kept at an even 37°C temperature and mixed 1 min before the turbidity measurement at each time point. The total simulation time was 8h.

The optical density was measured at the time points of 0.5, 3, 6 and 8 hours. The turbidity (optical density, OD) of growth media increases proportionally as the *Cl. perfringens* cell number and cell density increases.

The total gas production was measured at the end of the 8h simulation as an indicator of growth efficiency, since *Cl. perfringens* produces gas due to the active metabolism during exponential growth.

The student's t-test was used to compare the control treatment without TOFA against the saponified TOFA and digested saponified TOFA, and the two TOFA treatments against each other.

### Results

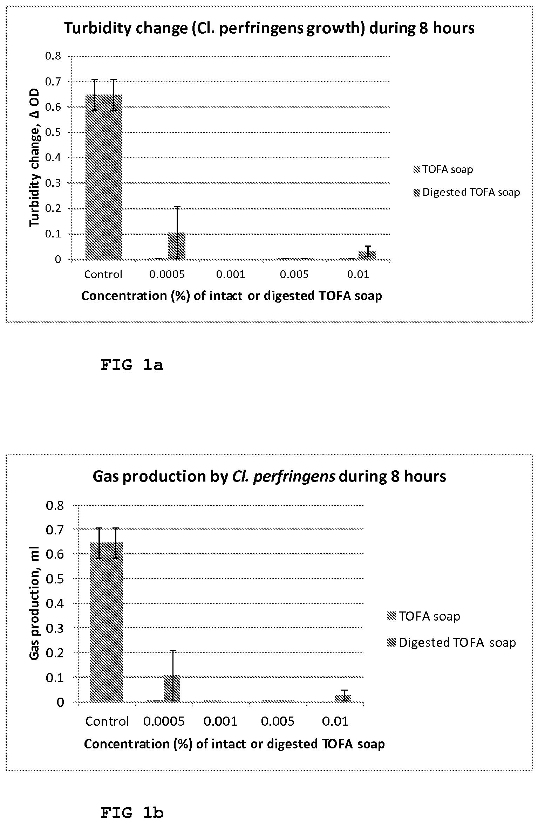

The results are illustrated in Figure 1a and 1b. The TOFA treatments very effectively and statistically significantly (p < 0.05) inhibited the growth of *Cl. perfringens* down to the concentration of 0.0005%, which was detected as the lack of turbidity change (Figure 1a) and the production of negligible amounts of gas (Figure 1b). The concentration 0.05% of TOFA, which is not shown in the figures, totally prevented the growth of *Cl. perfringens.*

The results show that saponified TOFA resists gastrointestinal digestion and maintains its efficacy against the growth of *Cl. perfringens.* This result shows that the saponified TOFA prevents or alleviates the onset of necrotic enteritis if given to broiler chicks or other species of poultry in the feed.

The experiment shows that the saponified TOFA is very effective against the growth of *Cl. perfringens,* and that most of its activity can resist gastrointestinal digestion.

### EXAMPLE 2.

### Pathogen inhibition test

This experiment was conducted to study the inhibition of *Cl. perfringens* by saponified TOFA with 7% resin acids. The saponification of the TOFA was conducted as described in Example 1. The efficiency of liquid and spray-dried TOFA soap was tested in a *Cl. perfringens* growth inhibition test that measures both the turbidity of clostridial culture medium as a result of an increased number of bacterial cells in a unit volume of the medium, and the cumulative gas production during the simulation.

Preparations of TOFA with 7% resin acids:
1. Liquid TOFA with a 20.4% dry matter content
2. Spray dried saponified TOFA

The spray-dried saponified TOFA was tested in the present trial in order to study the resistance of the product to drying and to detect any possible loss of efficacy due to the spray drying.

The efficiency of TOFA against the growth of *Cl. perfringens* was tested at eight concentrations of the dry matter of TOFA: 0% (Control), 0.01%, 0.005%, 0.001% 0.0005%, 0.0001%, 0.00005% and 0.00001%. Each treatment-concentration combination was replicated three times.

The simulation procedure and optical density and gas production measurements were conducted similarly as in Example 1.

The student's t-test was used to compare the control treatment without TOFA against the saponified TOFA and spray-dried saponified TOFA, and the two TOFA treatments against each other. The student's t-test p-values correspond to: ∼p < 0.1; *p < 0.05; **p < 0.01: ***p < 0.001; ****p < 0.0001.

### Results

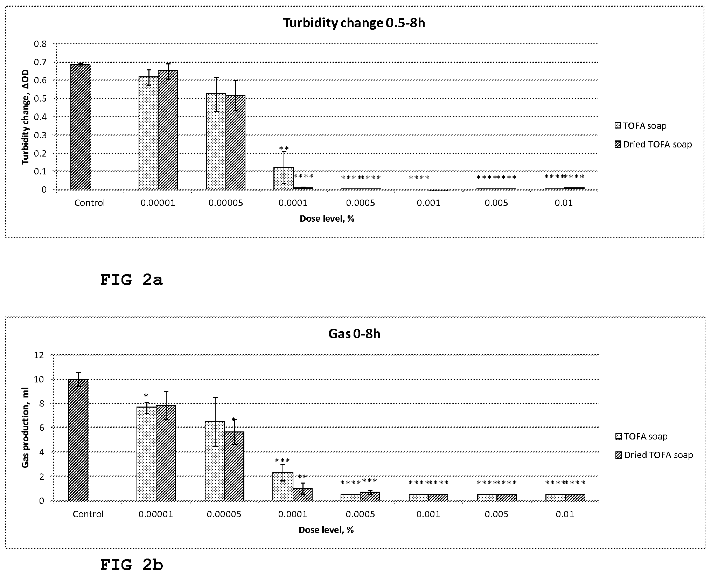

The results are illustrated in Figure 2a and 2b. The TOFA treatments very effectively and statistically significantly (p < 0.01) inhibited the growth of *Cl. perfringens* down to the concentration of 0.0001%, which was detected as the lack of turbidity change (Figure 2a) and the production of negligible amounts of gas (Figure 2b). The products at the concentrations of 0.00005% and 0.00001% had some inhibitory effect on the production of gas, indicating an anti-clostridial effect even at these low concentrations.

The results show that spray dried TOFA is as efficient against the growth of *Cl. perfringens* as liquid TOFA. Thus, the efficacy of the product is not impaired by spray drying. This result also shows that the saponified TOFA prevents or alleviates the onset of necrotic enteritis, if given to broiler chicks or other species of poultry in the feed.

This experiment suggests that the saponified TOFA is very effective against the growth of *Cl. perfringens,* as a liquid or spray dried product.

### EXAMPLE 3

### Pathogen inhibition test

This experiment was conducted to compare the efficacy of saponified TOFA with 8.5 % resin acids and competing products for their ability to inhibit the growth of pure cultures of three Gram-positive pathogens: *Clostridium perfringens, Staphylococcus aureus and Streptococcus suis in vitro.* The competing products were commercial natural plant extracts and a medium-chain fatty acid product. Plant extracts A-C are merchandised to have inhibitory effects against Gram+ pathogenic bacteria and they can be used e.g. in management of coccidiosis risk. The saponification of the TOFA was conducted as described in Example 1. Before the simulation, the bacteria were grown overnight as pure cultures in their specific growth medium. The bacterial cultures were used as inoculant in the experiment

Products and product doses are presented in Table 1.

| Product | Dose 1 (kg/ton of feed) | Dose 2 (kg/ton of feed) |
|---|---|---|
| Saponified TOFA 8.5 % resin acids | 0.25 | 0.50 |
| Plant (Oregano) extract A | 0.25 | 0.50 |
| Plant extract B | 0.25 | 0.50 |
| Plant extract C | 0.50 | 1.00 |
| Medium chain fatty acid product (MCFA) | 0.25 | 0.50 |

The test products were first weighed into the glass bottles. 15 ml of bacterial growth medium was added. Bottles for *Cl. perfringens* and *S. aureus* were prepared in anaerobic glove box, while the bottles for *S. suis* and *B. cereus* were prepared in aerobic environment. Next, the bottles were enclosed with air-tight stoppers to ensure anaerobic conditions throughout the experiment for *Cl. perfringens* and *S. aureus.* A needle was pushed through the stoppers of the two aerobic bacteria to ensure oxygen supply for the culture. 150 ml of bacterial culture was added into each bottle to act as an inoculum (1% of the volume). The simulation time was calculated starting from the time of inoculating each vessel. During the simulation, the bottles were kept at 37°C temperature in a constant, slow shaking for eight hours. Optical density was measured at the time points of 0, 2, 4, 6 and 8 hours. The turbidity (optical density, OD) of growth media increases proportionally as bacterial cell density increases.

Each product was tested at two concentrations and three replicates per concentration. Dose 2 was the recommended dose of the commercial products. Each product concentration had also a control vessel into which microbes were not included (one replicate/product/dose). These treatments controlled for any potential increase in the cloudiness that the test products may have induced into the growth medium during the simulation time. The total number of simulation vessels was 123 per bacterial species.

### Results

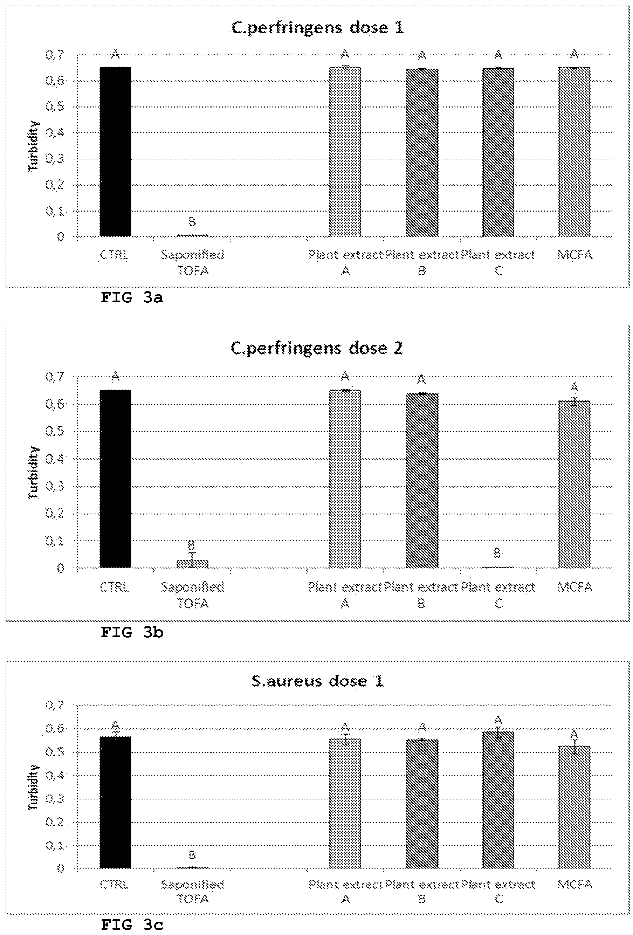

The results are illustrated in Figures 3a to 3f. The saponified TOFA of the invention totally inhibited the growth of *Cl. perfringens* at both product levels at the 8-hour time point (Figure 3a and 3b).

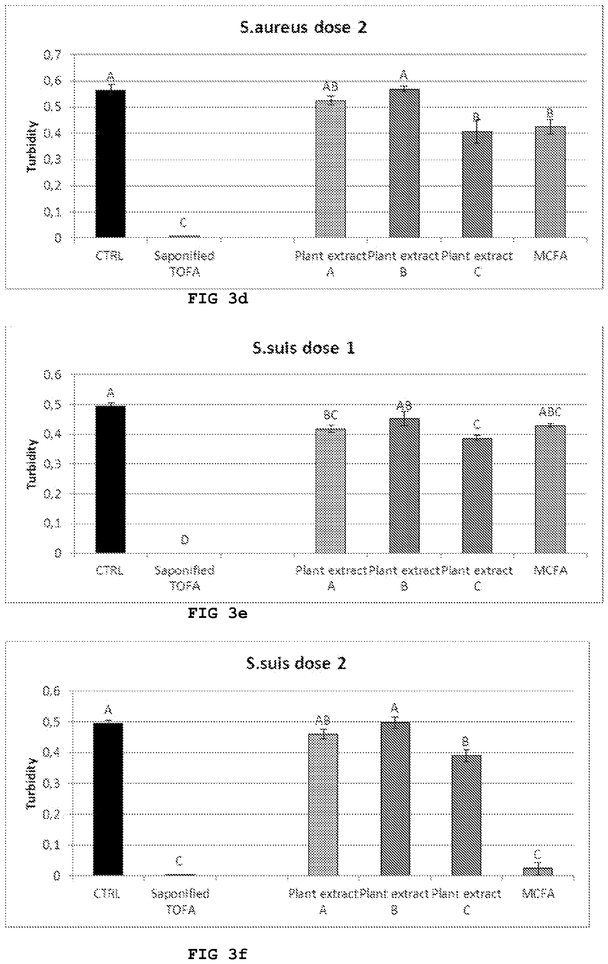

*S. aureus* was not able to grow at all in the presence of saponified TOFA at the studied concentrations, while the other products showed no inhibition at dose 1 (Figure 3c). Two of the other products showed partial inhibition at product dose 2 (Figure 3d).

Saponified TOFA fully prevented the growth of *Streptococcus suis* during the 8-hour simulation at both product doses (Figure 3e and 3f). At dose 2, MCFA product efficiently inhibited the growth of *S. suis* at the 8-hour time point (Figure 3f).

The experiment shows that the saponified TOFA is much more effective against the growth of *Cl. perfringens, Staphylococcus aureus and Streptococcus suis* as the commercial plant extracts A-C and MCFA claiming inhibitory effects against Gram+ pathogenic bacteria.

## Claims

1. A tall oil fatty acid comprising 1-10% (w/w) resin acids which is modified by saponification for use in the prevention of adverse effects on the digestive tract or health of an animal caused by growth of harmful bacteria in the animal digestive tract and/or in the prevention of intestinal disorders.

2. The tall oil fatty acid which is modified by saponification for use according to claim 1 , **characterized in that** it comprises 2-9 % (w/w) resin acids.

3. The tall oil fatty acid which is modified by saponification for use according to any of preceding claims 1 -2, **characterized in that** it comprises 5-9% (w/w) resin acids.

4. The tall oil fatty acid which is modified by saponification for use according to any of preceding claims 1 - 3, **characterized in that** it comprises 91-98% (w/w) fatty acids.

5. The tall oil fatty acid which is modified by saponification for use according to any of preceding claims 1 - 4, **characterized in that** it is dried.

6. Feed supplement comprising the tall oil fatty acid comprising 1-10% (w/w) resin acids which is modified by saponification for use in the prevention of adverse effects on the digestive tract or health of an animal caused by growth of harmful bacteria and/or in the prevention of intestinal disorders.

7. The feed supplement according to claim 6, **characterized in that** the tall oil fatty acid which is modified by saponification comprises 2-9 % (w/w), most preferably 5-9% (w/w) resin acids.

8. The feed supplement according to any of preceding claims 6 - 7, **characterized in that** the tall oil fatty acid which is modified by saponification is dried.

9. The feed supplement according to any of preceding claims 6 - 8, **characterized in that** the tall oil fatty acid which is modified by saponification is absorbed into a carrier material.

10. A feed composition comprising the feed supplement according to any of preceding claims 6 - 9 for use in the prevention of adverse effects on the digestive tract or health of an animal caused by growth of harmful bacteria in the animal digestive tract and/or in the prevention of intestinal disorders.

11. The feed composition for use according to claim 10, **characterized in that** it comprises said feed supplement in an amount of 0.00001 - 0.5 % (w/w), preferably 0.0005 - 0.1 % (w/w), most preferably 0.001 - 0.01 % (w/w) of the dry weight of the total amount of feed.

## Patentansprüche

1. Tallölfettsäure, die durch Verseifung modifiziert ist, umfassend 1-10 % (Gew./Gew.) Harzsäuren, zur Verwendung bei der Prävention von negativen Wirkungen auf den Verdauungstrakt oder die Gesundheit von einem Lebewesen, verursacht durch Wachstum von schädlichen Bakterien in dem Verdauungstrakt des Lebewesens und/oder bei der Prävention von Darmbeschwerden.

2. Tallölfettsäure, die durch Verseifung modifiziert ist, zur Verwendung nach Anspruch 1, **dadurch gekennzeichnet, dass** sie 2-9 % (Gew./Gew.) Harzsäuren umfasst.

3. Tallölfettsäure, die durch Verseifung modifiziert ist, zur Verwendung nach einem der vorangehenden Ansprüche 1-2, **dadurch gekennzeichnet, dass** sie 5-9 % (Gew./Gew.) Harzsäuren umfasst.

4. Tallölfettsäure, die durch Verseifung modifiziert ist, zur Verwendung nach einem der vorangehenden Ansprüche 1-3, **dadurch gekennzeichnet, dass** sie 91-98 % (Gew./Gew.) Fettsäuren umfasst.

5. Tallölfettsäure, die durch Verseifung modifiziert ist, zur Verwendung nach einem der vorangehenden Ansprüche 1-4, **dadurch gekennzeichnet, dass** sie getrocknet ist.

6. Futterergänzung, umfassend die Tallölfettsäure, die durch Verseifung modifiziert ist, umfassend 1-10 % (Gew./Gew.) Harzsäuren, zur Verwendung bei der Prävention von negativen Wirkungen auf den Verdauungstrakt oder die Gesundheit von einem Lebewesen, verursacht durch Wachstum von schädlichen Bakterien und/oder bei der Prävention von Darmbeschwerden.

7. Futterergänzung nach Anspruch 6, **dadurch gekennzeichnet, dass** die Tallölfettsäure, die durch Verseifung modifiziert ist, 2-9 % (Gew./Gew.), besonders bevorzugt 5-9% (Gew./Gew.), Harzsäuren umfasst.

8. Futterergänzung nach einem der vorangehenden Ansprüche 6-7, **dadurch gekennzeichnet, dass** die Tallölfettsäure, die durch Verseifung modifiziert ist, getrocknet ist.

9. Futterergänzung nach einem der vorangehenden Ansprüche 6-8, **dadurch gekennzeichnet, dass** die Tallölfettsäure, die durch Verseifung modifiziert ist, in einem Trägermaterial absorbiert ist.

10. Futterzusammensetzung, umfassend die Futterergänzung nach einem der vorangehenden Ansprüche 6-9 zur Verwendung bei der Prävention von negativen Wirkungen auf den Verdauungstrakt oder die Gesundheit von einem Lebewesen, verursacht durch Wachstum von schädlichen Bakterien in dem Verdauungstrakt des Lebewesens und/oder bei der Prävention von Darmbeschwerden.

11. Futterzusammensetzung zur Verwendung nach Anspruch 10, **dadurch gekennzeichnet, dass** sie die Futterergänzung in einer Menge von 0,00001 - 0,5 % (Gew./Gew.), vorzugsweise 0,0005 - 0,1 % (Gew./Gew.), besonders bevorzugt 0,001 - 0,01 % (Gew./Gew.), auf das Trockengewicht der Gesamtmenge des Futters umfasst.

## Revendications

1. Un acide gras de tall-oil comprenant 1 à 10% (p/p) d'acides résiniques qui est modifié par saponification pour une utilisation pour la prévention d'effets indésirables sur l'appareil digestif ou sur la santé d'un animal engendrés par la croissance de bactéries nocives dans l'appareil digestif de l'animal et/ou pour la prévention de troubles intestinaux.

2. L'acide gras de tall-oil qui est modifié par saponification pour une utilisation selon la revendication 1, **caractérisé en ce qu'**il comprend de 2 à 9% (p/p) d'acides résiniques.

3. L'acide gras de tall-oil qui est modifié par saponification pour une utilisation selon l'une quelconque des revendications 1 à 2 précédentes, **caractérisé en ce qu'**il comprend de 5 à 9% (p/p) d'acides résiniques.

4. L'acide gras de tall-oil qui est modifié par saponification pour une utilisation selon l'une quelconque des revendications 1 à 3 précédentes, **caractérisé en ce qu'**il comprend de 91 à 98% (p/p) d'acides gras.

5. L'acide gras de tall-oil qui est modifié par saponification pour une utilisation selon l'une quelconque des revendications 1 à 4 précédentes, **caractérisé en ce qu'**il a été séché.

6. Un complément alimentaire, comprenant l'acide gras de tall-oil comprenant 1 à 10% (p/p) d'acides résiniques qui est modifié par saponification pour une utilisation pour la prévention d'effets indésirables sur l'appareil digestif ou sur la santé d'un animal engendrés par la croissance de bactéries nocives dans l'appareil digestif de l'animal et/ou pour la prévention de troubles intestinaux.

7. Le complément alimentaire selon la revendication 6, **caractérisé en ce que** l'acide gras de tall-oil qui est modifié par saponification comprend de 2 à 9% (p/p), plus préférablement 5 à 9% (p/p) d'acides résiniques.

8. Le complément alimentaire selon l'une quelconque des revendications 6 ou 7 précédentes, **caractérisé en ce que** l'acide gras de tall-oil qui est modifié par un par saponification a été séché.

9. Le complément alimentaire selon l'une quelconque des revendications 6 à 8 précédentes, **caractérisé en ce que** l'acide gras de tall-oil qui est modifié par saponification est absorbé dans un matériau de support.

10. Une composition alimentaire comprenant le complément alimentaire selon l'une quelconque des revendications 6 à 9 précédentes, pour une utilisation pour la prévention d'effets indésirables sur l'appareil digestif ou sur la santé d'un animal dû à la croissance de bactéries nocives dans l'appareil digestif de l'animal et/ou pour la prévention de troubles intestinaux.

11. La composition alimentaire pour une utilisation selon la revendication 10, **caractérisée en ce qu'**elle comprend ledit complément d'alimentation en une quantité de 0,00001 à 0,5% (p/p), de préférence de 0,0005 à 0,1% (p/p), plus préférablement de 0,001 à 0,01 % (p/p) du poids sec de la quantité totale d'aliments.
